# EUROPÄISCHE PATENTSCHRIFT

(11) **EP 2 042 104 B1**
(45) Veröffentlichungstag und Bekanntmachung des Hinweises auf die Patenterteilung: **26.10.2011**
(21) Anmeldenummer: 07019216.6
(22) Anmeldetag: 29.09.2007
(51) Int. Cl.: A61B 17/04

(54) **Chirurgisches Nahtinstrument**
Surgical suturing instrument
Instrument de suture chirurgical

(43) Veröffentlichungstag der Anmeldung: 01.04.2009
(73) Patentinhaber: Richard Wolf GmbH, 75438 Knittlingen (DE)
(72) Erfinder: Prestel, Stephan, 76287 Rheinstetten-Mörsch (DE); Moser, Florian, Dipl.-Ing., 75015 Bretten (DE); Weber, Rolf, 76703 Kraichtal (DE)
(74) Vertreter: Hemmer, Arnd

(56) Entgegenhaltungen:
- WO-A-2004/082725
- US-A1- 2004 243 135
- US-A1- 2007 118 150
- US-A1- 2007 225 735
- US-B1- 6 723 107

## Beschreibung

Die Erfindung betrifft ein chirurgisches Nahtinstrument gemäß den im Oberbegriff des Anspruchs 1 angegebenen Merkmalen.

Nahtinstrumente der in Rede stehenden Art ermöglichen es, das zu vernähende Gewebe, wie z. B. Sehnen mit einem Zangenmaul zu ergreifen und anschließend mit einer in dem Nahtinstrument verschiebbar angeordneten Nadel zu durchstechen, wobei ein Faden mit der Nadel durch das Gewebe transportiert wird.

Solche Nahtinstrumente sind aus US 2005/0288690 A1 sowie DE 103 05 797 A1 bekannt. Die dort beschriebenen Nahtinstrumente weisen jeweils einen Schaft auf, an dessen distalen Ende ein Zangenmaul mit einem feststehenden und einem schwenkbaren Zangenmaulteil angeordnet ist. An dem feststehenden Zangenmaulteil ist ein gekrümmter Führungskanal ausgebildet, der an einer dem schwenkbaren Zangenmaulteil gegenüberliegenden Seite des feststehenden Zangenmaulteils mündet. Korrespondierend zur Lage der Mündung des Führungskanals weist das schwenkbare Zangenmaulteil eine Durchbrechung auf, durch die die Nadel nach dem Durchstechen von in dem Zangenmaul gehaltenem Gewebe geführt wird.

Das Nahtinstrument nach US 2005/0288690 A1 weist eine Nadel auf, bei der an einer Längsseite eine seitliche Nut zur Aufnahme eines Fadens vorgesehen ist. Korrespondierend zur Lage der Nadel in dem Führungskanal des feststehenden Zangenmaulteils ist an diesem Zangenmaulteil ein quer zur Längsausdehnung des Zangenmaulteils ausgerichteter Schlitz vorgesehen, der eine offene Verbindung zu dem Führungskanal schafft. Über diesen Schlitz kann der Faden in die seitliche Nut der Nadel eingelegt werden. Ein Nachteil dieser Ausgestaltung ist es, dass sich der Faden bei ungenauer Anordnung in der an der Nadel ausgebildeten Nut in dem sich distalseitig der Nut anschließenden Bereich des Führungskanals zwischen der Kanalwandung und der Nadel verklemmen kann; sodass das Nahtinstrument nicht mehr einsatzfähig ist. Weiter nachteilig ist bei diesem Instrument die Tatsache, dass die Nadel über einen sich in dem Schaft über die gesamte Schaftlänge erstreckenden Zufuhrkanal zu dem Führungskanal des feststehenden Zangenmaulteils geschoben werden muss. Dies ist insbesondere dann schwierig, wenn gekrümmte Nadeln verwendet werden, die an die Krümmung des Führungskanals angepasst sind.

Bei dem aus DE 103 05 797 A1 bekannten Nahtinstrument ist es nicht erforderlich, die Nadel durch den gesamten Schaft zu dem Führungskanal des feststehenden Zangenmaulteils zu transportieren. Dieses Nahtinstrument weist proximalseitig des feststehenden Zangenmaulteils einen Längsschlitz auf, der mit dem Führungskanal des Zangenmaulteils kommuniziert. Allerdings ist auch hier der Einsatz von gekrümmten Nadeln problematisch, da diese nur schwer in den Längsschlitz einführbar sind. Ein weiterer Nachteil dieses Nahtinstruments ist es, dass nur Nadeln verwendet werden können, an denen ein Faden befestigt ist. Dies führt dazu, dass eine Vielzahl Nadel-Faden-Kombinationen für unterschiedliche Nähaufgaben bereitgehalten werden muss.

Grundsätzlich kann festgestellt werden, dass die bislang bekannten chirurgischen Nahtinstrumente im Hinblick auf das Einsetzen der Nadel schwierig zu bedienen sind und/oder Nachteile hinsichtlich ihrer Funktionssicherheit aufweisen.

Vor diesem Hintergrund liegt die Aufgabe der Erfindung darin, ein chirurgisches Nahtinstrument und solche Komponenten für ein chirurgisches Nahtinstrument bereitzustellen, die bei einem einfachen Aufbau eine verbesserte Funktionsfähigkeit und Bedienbarkeit des Nahtinstruments gewährleisten.

Diese Aufgabe wird durch ein chirurgisches Nahtinstrument mit den im Anspruch 1 angegebenen Merkmalen, einen Nadelhalter mit den in Anspruch 7 angegebenen Merkmalen sowie durch eine Nadel mit den in Anspruch 13 angegebenen Merkmalen gelöst. Vorteilhafte Weiterbildungen der Erfindung ergeben sich aus den Unteransprüchen, der nachfolgenden Beschreibung sowie den Zeichnungen.

Das erfindungsgemäße chirurgische Nahtinstrument weist einen Schaft auf, an dessen distalen Ende ein Zangenmaul ausgebildet ist. Darüber hinaus weist das Nahtinstrument eine Nadelführung durch das Zangenmaul auf. Die Grundidee der Erfindung liegt darin, dass an dem Schaft proximalseitig an die Nadelführung anschließend eine Aufnahme zum lösbaren Befestigen eines Nadelhalters ausgebildet ist.

Diese Aufnahme zum lösbaren Befestigen des Nadelhalters ist derart ausgebildet, dass ein zur Aufnahme einer Nadel vorgesehener Nadelhalter an ihr schnell befestigt bzw. schnell von ihr gelöst werden kann. Die Aufnahme kann beispielsweise an einer Außenseite des Schaftes als eine Ausnehmung an dem Schaft ausgebildet sein. An ihrem distalen Ende weist die Aufnahme typischerweise einen offenen Zugang zu der Nadelführung auf.

Derart ausgebildet ermöglicht es die Erfindung, das Nahtinstrument einfach und schnell mit einer Nadel zu bestücken. Hierzu kann räumlich getrennt von dem Nahtinstrument in dem Nadelhalter eine Nadel angeordnet werden und der Nadelhalter anschließend mitsamt der Nadel in oder an der an dem Schaft ausgebildeten Aufnahme befestigt werden. Da der Nadelhalter, wenn er sich an dem Schaft ausgebildeten Aufnahme befindet, proximalseitig der Nadelführung, vorzugsweise direkt an diese anschließend angeordnet ist, kann die Nadel unter Verwendung geeigneter Betätigungsmittel über eine verhältnismäßig kurze Wegstrecke und günstigstenfalls direkt von dem Nadelhalter in die Nadelführung des Nahtinstruments bewegt werden. Darüber hinaus macht es diese Ausgestaltung auch möglich, Nadelhalter mit einer darin angeordneten Nadel vorkonfektioniert zur Verfügung zu stellen, was einen deutlichen Zeitgewinn bei der Einsatzvorbereitung des erfindungsgemäßen Nahtinstruments bedeutet. Dann kann ein umständliches Einsetzen einer recht kleinen Nadel in das Instrument vermieden werden. Stattdessen wird lediglich ein solch vorkonfektionierter Nadelhalter mit Nadel an das Nahtinstrument angesetzt. Ein weiterer Vorteil solcher vorbestückter Nadelhalter ist darin zu sehen, dass die beim Bestücken des Nadelhalters bestehende Verletzungsgefahr für den Benutzer des erfindungsgemäßen Nahtinstruments entfällt.

Vorteilhaft ist bei dem erfindungsgemäßen Nahtinstrument ein Nadelhalter vorgesehen, der eine Nadelführung zur Aufnahme und Führung einer Nadel aufweist und derart lösbar an der Aufnahme des Schaftes befestigt ist, dass sich die Nadelführung im Nadelhalter an die Nadelführung durch dass Zangenmaul anschließt.

Dementsprechend ist der Nadelhalter so ausgebildet und so an der Aufnahme des Schaftes angeordnet, dass die an dem Nadelhalter ausgebildete Nadelführung eine proximale Verlängerung der Nadelführung durch das Zangenmaul darstellt. Der Nadelhalter bildet bevorzugt einen Teil des Schaftes. Hierzu kann die an dem Schaft vorgesehene Aufnahme für den Nadelhalter von einer Ausnehmung an dem Schaft gebildet sein, wobei der Nadelhalter eine zu der Ausnehmung korrespondierende Form aufweist, so dass der Nadelhalter und der Schaft im zusammengebauten Zustand eine fluchtende Außenfläche bilden. Hierdurch kann eine besonders schlanke Bauform des erfindungsgemäßen Nahtinstruments verwirklicht werden.

Zweckmäßigerweise sind an dem Schaft und/oder dem Nadelhalter Befestigungsmittel zum lösbaren Befestigen des Nadelhalters an der Aufnahme an dem Schaft vorgesehen. Diese Befestigungsmittel sind bevorzugt so angeordnet, dass sie den Nadelhalter sowohl im Bereich seines distalen als auch im Bereich seines proximalen Endes zumindest in einer Richtung festlegen, die von dem Schaft radial auf den Nadelhalter weist. Hierbei ist die Art, wie diese Befestigungsmittel den Nadelhalter an der an dem Schaft vorgesehen Aufnahme festlegen, grundsätzlich beliebig. So können schaft- und nadelhalterseitig Befestigungsmittel vorgesehen sein, die z. B zur Bildung einer Steck- oder Rastverbindung zwischen Aufnahme und Nadelhalter ausgebildet sind oder die Befestigungsmittel können magnetisch wirkend ausgebildet sein.

Bevorzugt sind die Befestigungsmittel nach Art eines Bajonettverschlusses ausgestaltet. Dies ermöglicht ein besonders einfaches und schnelles Befestigen bzw. Lösen des Nadelhalters an bzw. von der an dem Schaft ausgebildeten Aufnahme. Hierzu sind an dem Schaft und hier vorzugsweise an der Aufnahme sowie an dem Nadelhalter Befestigungsmittel vorgesehen, die miteinander zusammengesteckt werden können, wobei sie bei einer anschließenden relativen Drehbewegung des Nadelhalters gegenüber dem Schaft einen Formschluss bilden, welcher den Nadelhalter in radialer Richtung in Bezug auf die Längsachse des Schaftes, bevorzugt jedoch auch in axialer und ggf. tangentialer Richtung sichert.

Zur Bildung einer solchen bajonettverschlussartigen Befestigung des Nadelhalters an der Aufnahme des Schaftes ist an dem Schaft, bevorzugt an der Aufnahme des Schaftes, vorteilhafterweise ein Vorsprung ausgebildet, der quer, bevorzugt radial, zu einer Längsachse des Schaftes ausgerichtet ist. Hierbei ist der Endbereich des Vorsprungs in zumindest einer Richtung quer zur Längsausdehnung des Vorsprungs erweitert.

Dementsprechend kragt das freie Ende des Vorsprungs in zumindest eine Richtung von dem Vorsprung radial nach außen. Diese Ausbildung ermöglicht es bei korrespondierender Ausgestaltung eines nadelhalterseitigen Befestigungsmittels, dieses Befestigungsmittel auf den Vorsprung aufzusetzen und den Nadelhalter anschließend derart relativ zu dem Schaft zu verdrehen bzw. zu verschwenken, dass die Erweiterung des Vorsprungs einen Bereich des nadelhalterseitigen Befestigungsmittels hintergreift, wodurch der Nadelhalter in Aufsetzrichtung des nadelhalterseitigen Befestigungsmittels quer zur Längsachse des Schaftes formschlüssig festgelegt wird.

Eine weitere vorteilhafte Ausgestaltung des erfindungsgemäßen Nahtinstruments sieht vor, dass an einem Maulteil des Zangenmauls eine Fadenführung ausgebildet ist, die die Nadelführung durch das Zangenmaul kreuzt. Hierbei verläuft die Fadenführung zwischen den beiden Maulteilen des Zangenmauls und bevorzugt direkt oberhalb einer an einem Maulteil vorgesehenen Öffnung der Nadelführung. Die Fadenführung ist zweckmäßigerweise derart ausgebildet, dass ein Faden schlaufenförmig über diese Öffnung der Nadelführung gelegt werden kann. Wird bei Einsatz des Nahtinstruments eine Nadel durch die Nadelführung des Zangenmauls bewegt, erfasst das distale Ende der Nadel den Faden und durchstößt zusammen mit dem oben aufliegenden Faden das Gewebe, das in dem Zangenmaul gehalten ist.

Um die Nadel von dem Nadelhalter über die Nadelführung durch das Zangenmaul bewegen zu können, weist das erfindungsgemäße Nahtinstrument zweckmäßigerweise eine Betätigungsstange auf, die im Bereich der Aufnahme für den Nadelhalter Eingriffsmittel zum Ineingrifftreten mit einer in dem Nadelhalter eingesetzten Nadel aufweist.

Diese Betätigungsstange ist bevorzugt in dem Schaft parallel zu dessen Längsachse bewegbar geführt und mit einer im Bereich des proximalen Endes des Schaftes angeordneten Handhabe betätigbar. Vorzugsweise im Bereich des distalen Endes ist an der Betätigungsstange ein Eingriffmittel bevorzugt in Form eines im Wesentlichen quer zur Längsausdehnung der Betätigungsstange ausgerichteten Vorsprungs oder einer quer zur Längsausdehnung der Betätigungsstange ausgerichteten Ausnehmung ausgebildet. Bei dieser Ausgestaltung weist eine in dem Nadelhalter eingesetzte Nadel zweckmäßigerweise eine zu einem betätigungsstangenseitig ausgebildeten Vorsprung korrespondierende Ausnehmung oder bei einer betätigungsstangenseitig ausgebildeten Ausnehmung einen korrespondierenden Vorsprung auf. Durch diesen formschlüssigen Eingriff kann die Nadel mittels der Betätigungsstange in der Nadelführung distalwärts und vorzugsweise auch danach wieder proximalwärts bewegt werden.
Die Erfindung betrifft auch einen Nadelhalter, der eine Nadelführung zur Aufnahme und Führung einer Nadel aufweist. Darüber hinaus ist der Nadelhalter zum lösbaren Befestigen an einer Aufnahme eines Schaftes eines chirurgischen Nahtinstruments ausgebildet. Insbesondere ist der erfindungsgemäße Nadelhalter zur Verwendung mit einem Nahtinstrument gemäß der vorangehenden Beschreibung ausgebildet.

Aus diesem Grund sind an dem Nadelhalter vorteilhafterweise Befestigungsmittel zu dessen lösbaren Befestigen an der Aufnahme an dem Schaft eines chirurgischen Nahtinstrument vorgesehen. Die Art dieser Befestigungsmittel ist grundsätzlich frei wählbar solange diese Befestigungsmittel komplementär zu ggf. an dem Schaft des chirurgischen Nahtinstrumentes ausgebildeten Befestigungsmitteln sind. Der Nadelhalter kann zur Mehrfachverwendung ausgebildet sein oder, wie es bevorzugt vorgesehen ist, zum einmaligen Gebrauch bestimmt sein und kann beispielsweise als ein Kunststoffspritzgussteil ausgebildet sein. Bevorzugt ist der Nadelhalter in dem erfindungsgemäßen chirurgischen Nahtinstrument angeordnet.

Die Nadelführung in dem Nadelhalter ist bevorzugt eine Nut, die sich in Längsrichtung des Nadelhalters erstreckt und die an einem distalen Ende des Nadelhalters endet. Hierbei kann sich die Nut über die gesamte Länge des Nadelhalters erstrecken. Vorteilhaft erstreckt sich die Nut allerdings nur über einen Teilbereich des Nadelhalters, sodass das proximale Ende der Nut verschlossen ist und auf diese Weise einen Anschlag für eine in dem Nadelhalter anzuordnende Nadel bildet. Diese Ausgestaltung ermöglicht es, eine Nadel reproduzierbar in dem Nadelhalter in einer bestimmten, durch das Anliegen des proximalen Nadelendes an dem Nutende definierten Position anzuordnen. Dementsprechend gewährleistet diese Ausgestaltung des Nadelhalters auch, Nadeln nach dem Anbringen des Nadelhalters an einem Nahtinstrument in diesem Nahtinstrument immer in einer festgelegten Position anzuordnen, in der beispielsweise an den Nadeln ausgebildete Eingriffsmittel mit Eingriffsmitteln eines in dem Nahtinstrument zum Bewegen der Nadel vorgesehenen Betätigungsmittel in Eingriff treten können.

Um ein Herausfallen der Nadel aus dem Nadelhalter möglichst zu verhindern, sind vorteilhaft Mittel vorgesehen, die die offene Seite der Nut zumindest abschnittsweise überdecken. Dementsprechend kann zumindest ein Bauteil vorgesehen sein, das derart an dem Nadelhalter angeordnet ist, dass es sich oberhalb der Nut und vorzugsweise im Wesentlichen quer zur Nut erstreckt. Bei diesem Bauteil kann es sich z. B. um einen Steg handeln, der quer über die Nut geführt ist. Auf diese Weise ist eine in der Nut befindliche Nadel in allen Richtungen quer zur Längsausdehnung der Nut gegen ein Herausfallen aus der Nut gesichert.

Bevorzugt ist im Bereich des proximalen Endes des Nadelhalters ein vorzugsweise schräg zur Längsausdehnung des Nadelhalters ausgerichtetes Langloch ausgebildet. Dieses Langloch verläuft vorzugsweise von einer im einbauten Zustand an einer an dem Nahtinstrument ausgebildeten Aufnahme für den Nadelhalter anliegenden Innenseite des Nadelhalters zu einer in diesem Zustand an dem Nahtinstrument radial außenliegenden Außenseite des Nadelhalters. Das Langloch ermöglicht die Aufnahme eines an der Aufnahme für den Nadelhalter an dem Nahtinstrument angeordneten Befestigungsmittels, das, wie bereits weiter oben beschrieben, einen normal zur Auflagefläche der Aufnahme ausgerichteten Vorsprung aufweist, wobei der Vorsprung an seinem freien Ende an zumindest einer Richtung quer zu seiner Längsausdehnung eine Erweiterung aufweist. Auf ein solches aufnahmeseitiges Befestigungsmittel wird der Nadelhalter derart aufgesetzt, dass sich die Erweiterung des aufnahmeseitigen Befestigungsmittels oberhalb des Langlochs befindet. Bei einer anschließenden Drehung des Nadelhalters relativ zu dem Schaft des Nahtinstruments kann die an dem aufnahmeseitigen Befestigungsmittel vorgesehene Erweiterung einen seitlich der Längskante des Langlochs gelegenen Bereich des Nadelhalters übergreifen, wodurch der Nadelhalter formschlüssig an der Aufnahme des Nahtinstruments festgelegt wird. Um zu verhindern, dass die Erweiterung des aufnahmeseitigen Befestigungsmittels außenseitig des Nadelhalters vorsteht, kann der Nadelhalter an dieser Außenseite zweckmäßigerweise einen Einstich aufweisen, in dem die Erweiterung des aufnahmeseitigen Befestigungsmittels zur Anlage kommt, ohne dabei die Außenseite des Nadelhalters zu überragen.

Die Erfindung betrifft ferner eine Nadel, die für den Einsatz in einem Nadelhalter sowie Nahtinstrument gemäß der vorangehenden Beschreibung vorgesehen ist. Diese Nadel wird von einem vorzugsweise geraden Metallstreifen gebildet, der ein distales und ein proximales Ende aufweist. An dem distalen Ende dieses Materialstreifens ist eine Schneidkante ausgebildet, die sich im Wesentlichen normal zur Bewegungsrichtung der Nadel oder ausgehend von einer sich normal zur Bewegungsrichtung erstreckenden Stirnkante erstreckt. Gemäß der Erfindung ist an der Stirn- oder Schneidkante eine sich quer zur Stirn- oder Schneidkante proximalwärts erstreckende Ausnehmung ausgebildet, die eine proximalseitige nichtschneidend ausgebildete Kante aufweist.

Diese Ausbildung der erfindungsgemäßen Nadel ermöglicht es in vorteilhafter Weise, einen Faden durch das zu vernähende Gewebe zu transportieren, wobei der Faden schlaufenförmig auf dem distalen Ende der Nadel aufliegt. Hierbei ist der Faden relativ zu der Nadel so zu positionieren, dass er auf der nichtschneidend ausgebildeten Kante des distalen Endes der Nadel zur Auflage kommt. Im Gegensatz zu den bislang bekannten Nadeln dieser Art, besteht nicht die Gefahr, dass der Faden durch die Schneide der Nadel beschädigt wird oder von dieser Schneide aufgespießt wird und so bei einem Zurückbewegen der Nadel aus dem zuvor durchstochenen Gewebe wieder mit zurückbewegt wird.

Die an der Stirn- bzw. Schneidkante ausgebildete Ausnehmung ist zweckmäßigerweise nur so breit ausgebildet, dass sie ein sicheres Einlegen eines Fadens in diese Ausnehmung gewährleisten kann. Die Form der Ausnehmung ist grundsätzlich beliebig solange sie eine ausreichend große nichtschneidend ausgebildete Aufnahmekante für einen Faden aufweist, die quer zur Bewegungsrichtung der Nadel ausgerichtet ist. Auch die Position der Ausnehmung an der Stirn- bzw. Schneidkante ist prinzipiell frei wählbar, solange gewährleistet ist, dass der Faden von dieser Ausnehmung ergriffen werden kann. So kann diese Ausnehmung an einem Randbereich dieser Schneidkante angeordnet sein und einen nichtschneidenden Absatz der Schneidkante bilden, wobei der Faden nur mit diesem nichtschneidenden Absatz in Kontakt kommt.

Bevorzugt ist an der Stirn- bzw. Schneidkante eine Nut ausgebildet, die die Schneidkante in zwei voneinander beabstandete Schneidkantenabschnitte teilt, wobei der Nutboden nichtschneidend ausgebildet ist. Dementsprechend bildet bei dieser Ausgestaltung die Nut, die vorzugsweise im Wesentlichen mittig in der Schneidkante angeordnet ist, die nicht schneidend ausgebildete Ausnehmung. Diese Ausgestaltung hat den Vorteil, dass der Faden gegen eine Bewegung quer zur Bewegungsrichtung der Nadel gesichert ist und so nicht von dem distalen Ende der Nadel seitlich abrutschen kann.

In vorteilhafter Weiterbildung ist die Nut derart ausgebildet, dass sich die Nutbreite in proximaler Richtung verjüngt. So kann die Nut beispielsweise keilförmig ausgebildet sein, wobei die Seitenkanten der Nut ausgehend von der Stirn- bzw. Schneidkante, an der diese Seitenkanten am weitesten voneinander beabstandet sind, in proximaler Richtung der Nadel schräg aufeinander zulaufen. Um das Durchdringen von Gewebe zu erleichtern, können die Seitenkanten der Nut in Ihrem distalen Endbereich schneidend ausgebildet sein, wobei der zur Aufnahme des Fadens dienende proximalseitige Bodenbereich der Nut allerdings nichtschneidend ausgebildet ist.

In einer bevorzugten Ausgestaltung verjüngt sich die Breite der an der Stirn- bzw. Schneidkante ausgebildeten Nut in proximaler Richtung zweistufig. Hierbei bilden die beiden Seitenwände der Nut in einem ersten distalen Abschnitt der Nut schneidende Kanten, wobei ein proximaler Abschnitt der Nut eine nichtschneidende Kante aufweist und eine Aufnahme für einen Faden bildet. Besonders vorteilhaft erstreckt sich die Nut über die gesamte Breite des distalen Nadelendes. Ausgehend von den beiden Längsseiten der Nadel können hierbei die Seitenkanten der Nut zunächst in einem verhältnismäßig stumpfen Winkel aufeinander zulaufen und in einem sich proximalseitig daran anschließenden Abschnitt der Nut spitzer aufeinander zulaufen. Die distalseitige im stumpfen Winkel aufeinander zulaufenden Kanten sind dabei schneidend ausgebildet und bilden die eigentlichen Schneidkanten. Auf diese Weise weist die Nadel bei dieser Ausgestaltung zwei schräg zur Bewegungsrichtung der Nadel ausgerichtete schneidende Kanten auf, die jeweils eine Spitze bilden. Bei dieser Ausführungsform erstreckt sich die Nut somit über die gesamte Breite der Stirnkante der Nadel und die Schneidkanten selber sind schräg verlaufend zu dieser Stirnkante ausgebildet. Es ist jedoch auch denkbar, das distale Ende der Nadel so auszugestalten, dass Teile der Schneidkante in Richtung der Stirnkante verlaufen und beispielsweise die Nut schmaler ausgebildet ist, sodass sie sich am distalen Ende nicht über die gesamte Breite der Stirnkante erstreckt.

Um die erfindungsgemäße Nadel mittels einer Betätigungsstange in eine Arbeitsstellung sowie in eine Ausgangsstellung bewegen zu können, weist die Nadel zweckmäßigerweise Mittel auf, die mit einer Betätigungsstange eines chirurgischen Nahtinstruments in Eingriff treten können. Hierbei kann es sich um an der Nadel ausgebildete Ausnehmungen und Vorsprünge handeln, die zur Bildung eines Formschlusses mit korrespondierenden an der Betätigungsstange vorgesehenen Eingriffsmitteln, wie sie weiter oben beschrieben sind, dienen. Bevorzugt weist die Nadel an einer Längsseite im Bereich ihres proximalen Endes eine quer zu Ihrer Längsausdehnung verlaufende Ausnehmung auf, in die ein an einer Betätigungsstange quer zu dessen Bewegungsrichtung ausgerichteter Vorsprung eingreifen kann.

Bevorzugt ist die erfindungsgemäße Nadel in der Nadelführung des erfindungsgemäßen Nadelhalters angeordnet.

Nachfolgend ist die Erfindung anhand eines in den Zeichnungen dargestellten Ausführungsbeispiels näher erläutert. Darin zeigen:
- Fig. 1: ein chirurgisches Nahtinstrument in einem Längsschnitt,
- Fig. 2: eine Schnittansicht entlang der Schnittlinie II-II in Fig. 1,
- Fig. 3: eine Einzelheit III aus Fig. 1 in vergrößerter Darstellung,
- Fig. 4: eine Seitenansicht des chirurgischen Nahtinstruments aus Fig. 1 mit einer ausgefahrenen Nadel,
- Fig. 5: eine Einzelheit IV aus Fig. 4 in vergrößerter Darstellung,
- Fig. 6: einen Nadelhalter mit darin angeordneter Nadel in perspektivischer Darstellung,
- Fig. 6a: eine Einzelheit VI aus Fig. 6 in vergrößerter Darstellung,
- Fig. 7: den distalen Endbereich des Instruments nach Fig. 1 mit dem Nadelhalter nach Fig. 6 in einer Explosionsdarstellung,
- Fig. 8: den distalen Endbereich des Instruments nach Fig. 1 mit dem daran angebrachten Nadelhalter nach Fig. 6,
- Fig. 9: einen Außenschaft für das chirurgische Instrument nach Fig. 1,
- Fig. 10: das Instrument nach Fig. 1 mit dem Außenschaft nach Fig. 10 in perspektivischer Explosionsdarstellung,
- Fig. 11: der distale Endbereich des Instruments nach Fig. 1 in einer ersten Arbeitsstellung,
- Fig. 12: der distale Endbereich des Instruments nach Fig. 1 in einer zweiten Arbeitsstellung,
- Fig. 13: der distale Endbereich des Instruments nach Fig. 1 in einer dritten Arbeitsstellung und
- Fig. 14: der distale Endbereich des Instruments nach Fig. 1 in einer vierten Arbeitsstellung,
- Fig. 15: eine Seitenansicht des chirurgischen Nahtinstrument in einer weiteren Ausführungsform.

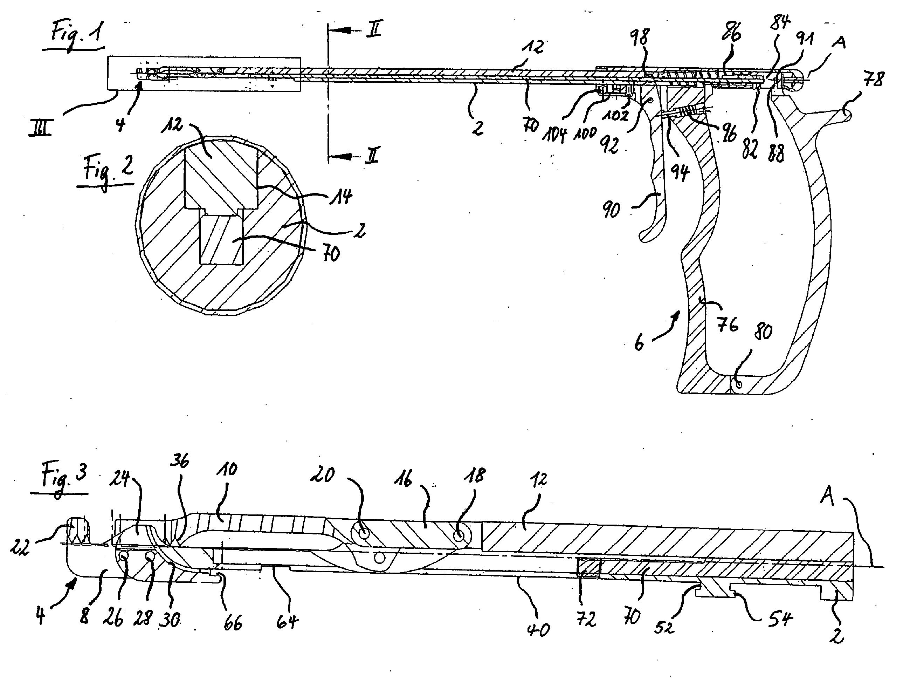

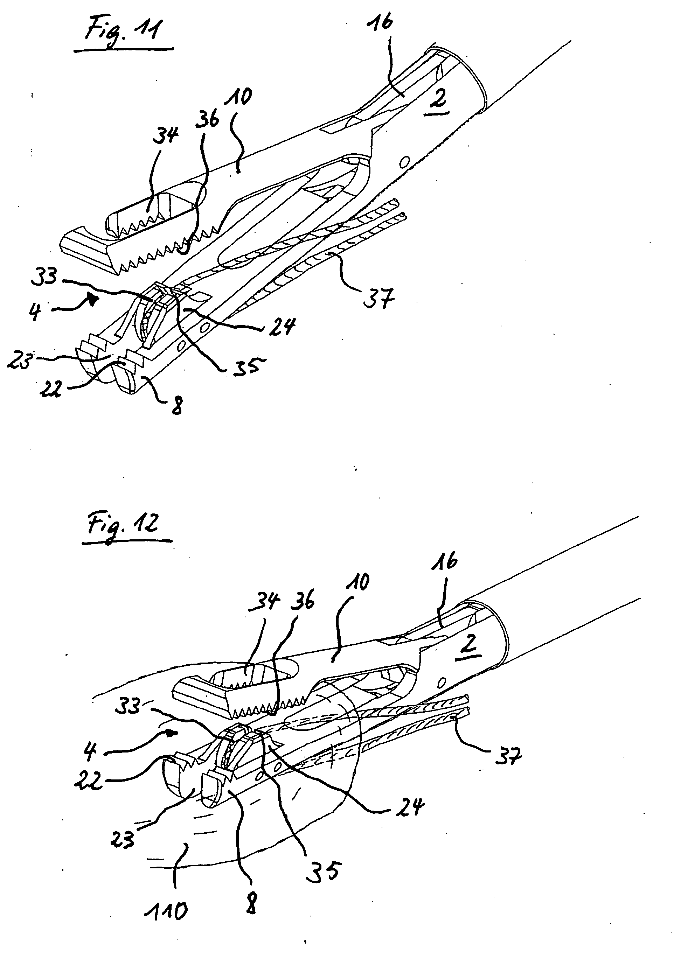

Das in Fig. 1 dargestellte chirurgische Nahtinstument weist einen Schaft 2 auf, an dessen distalen Ende ein Zangenmaul 4 zum Festhalten von Körpergewebe 110 (siehe Fig. 12) ausgebildet ist. An dem proximalen Ende des Schaftes 2 ist eine Handhabe 6 zum Halten und Bedienen des Nahtinstruments angeordnet. Das Zangenmaul 4 weist ein erstes feststehend an dem Schaft 2 angeordnetes Maulteil 8 und ein gegenüber dem Maulteil 8 schwenkbar an dem Schaft 2 angelenktes zweites Maulteil 10 auf.

Die Betätigung des Zangenmauls 4 erfolgt über eine Betätigungsstange 12, die in einer an dem Schaft 2 ausgebildeten Nut 14 parallel zu einer Längsachse A des Schaftes 2 verschiebbar geführt ist. Hierzu ist das Maulteil 10 über ein Gelenkteil 16 mit der Betätigungsstange 12 bewegungsgekoppelt, wobei das Gelenkteil 16 über einen Stift 18 an dem distalen Ende der Betätigungsstange 12 und über einen Stift 20 an dem proximalen Ende des Maulteils 10 angelenkt ist.

Das Maulteil 8 weist, wie insbesondere den Fig. 11 bis 14 zu entnehmen ist, distalseitig einen gezahnt ausgebildeten Haltebereich 22 auf. Ausgehend von dem distalen Ende dieses Haltebereichs 22 erstreckt sich ein Schlitz 23 in Längsrichtung des Schaftes 2 proximalwärts. In diesem Schlitz 23 ist proximalseitig des Haltebereichs 22 ein Nadelführungsbauteil 24 angeordnet, das über zwei Stifte 26 und 28 an dem Maulteil 8 befestigt ist. Das Nadelführungsbauteil bildet eine höckerartige Erhebung, die sich in Richtung des zweiten Maulteils 10 erstreckt. In dem Nadelführungsbauteil 24 ist ein gekrümmter Führungskanal 30 ausgebildet, der Teil einer durch das Zangenmaul 4 geführten Nadelführung für eine Nadel 32 ist. Dieser Führungskanal 30 mündet an einer dem zweiten Maulteil 10 zugewandten Seite der Erhebung des Nadelführungsbauteils 24.

An der dem zweiten Maulteil 10 zugewandten Außenseite der Erhebung des Nadelführungsbauteils 24 ist eine in Richtung der Längsausdehnung des Schaftes 2 verlaufende Nut 33 ausgebildet, die eine an der Oberseite der Erhebung des Nadelführungsbauteils 24 angeordnete Mündung 35 des Führungskanals 30 kreuzt. Diese Nut 33 dient zur Führung eines Fadens 37 über dem Führungskanal 30.

Zur Aufnahme der höckerartigen Erhebung des Nadelführungsbauteils 24 sowie insbesondere zur Durchführung der Nadel 32 weist das Maulteil 10 eine Durchbrechung 34 auf. Diese Durchbrechung 34 ist im Bereich eines an dem distalen Ende des Maulteils 10 verzahnt ausgebildeten Haltebereichs 36 angeordnet. Über einen Schlitz ist die Durchbrechung 34 auch von einer Längsseite des Maulteils 10 zugänglich.

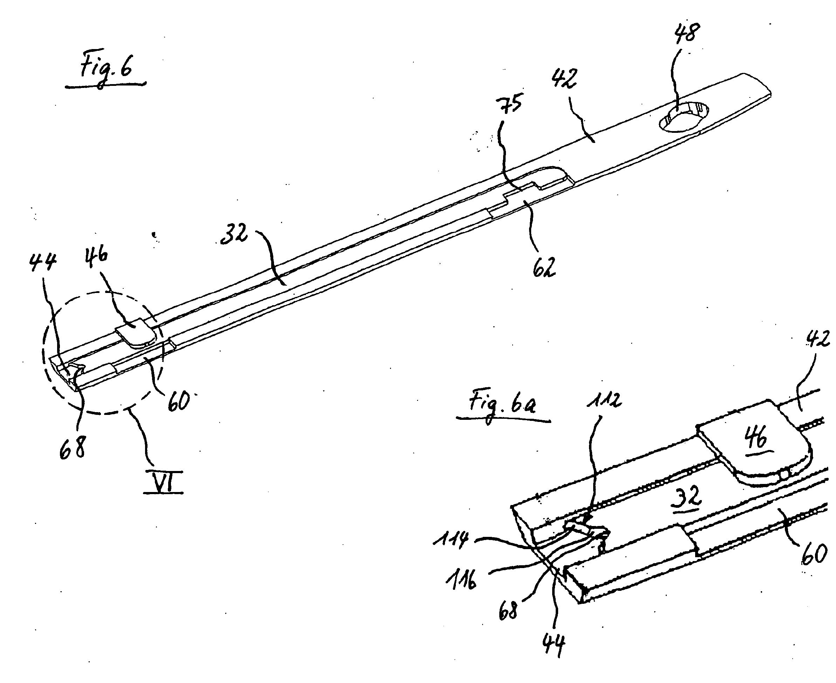

Proximalseitig des Maulteils 8 weist der Schaft 2 des chirurgischen Nahtinstruments eine längliche Abflachung bzw. Ausnehmung 40 auf. Diese Ausnehmung 40 bildet eine Aufnahme für den in Fig. 6 dargestellten Nadelhalter 42, der zum einmaligen Gebrauch vorgesehen ist. Der Nadelhalter 42 weist eine mit der an dem Schaft 2 ausgebildeten Ausnehmung 40 korrespondierende Form auf, so dass seine Außenseite in dem am Schaft 2 angebrachten Zustand mit der Außenseite des Schaftes 2 fluchtet. An einer dem Schaft 2 im angebrachten Zustand zugewandten Innenseite ist an dem Nadelhalter 42 ausgehend von dessen distalen Ende eine Nut 44 ausgebildet, die eine Nadelführung zur Aufnahme und Führung einer Nadel 32 bildet. Wird der Nadelhalter 42 proximalseitig an das Nadelführungsbauteil 24 angesetzt, fluchtet diese Nut 44 im eingebauten Zustand des Nadelhalters 42 mit dem an dem Nadelführungsbauteil 24 ausgebildeten Führungskanal 30, so dass eine Nadel 32 von der Nut 44 direkt in den Führungskanal 30 des Nadelführungsbauteils 24 und von dort durch das Zangenmaul 4 verschoben werden kann. Das proximale Ende der Nut 44 ist geschlossen und bildet einen Anschlag für die Nadel 32. Die Abmessungen der Nut 44 sind an die Abmessungen der Nadel 32 angepasst. So entspricht die Breite der Nut 44 im Wesentlichen der Breite der Nadel 32, wobei die Länge der Nut 44 so bemessen ist, dass die Nadel 32 vollständig in der Nut 44 aufgenommen werden kann. Oberhalb der Nut 44 ist im Bereich des distalen Endes des Nadelhalters 42 ein Bauteil in Form eines Stegs 46 angeordnet, das sich ausgehend von der die Nut 44 begrenzenden Wandung des Nadelhalters 42 quer zur Ausrichtung der Nut 44 im Wesentlichen vollständig über diese Nut 44 hinweg erstreckt.

Die in dem Nadelhalter 42 angeordnete Nadel 32 wird von einem geraden Metallstreifen gebildet. Das distale Ende der Nadel 32 bildet eine Schneidkante 112. An dieser Schneidkante 112 ist eine Nut 68 ausgebildet, die die Schneidkante 112 in zwei voneinander beabstandete Schneidkantenabschnitte teilt. Die Breite der Nut 68 verjüngt sich ausgehend von der Schneidkante 112 in proximaler Richtung zweistufig. Hierbei laufen die beiden Seitenkanten der Nut 68 in einem ersten distalen Bereich 114 zunächst verhältnismäßig stumpf aufeinander zu. In einem sich an den ersten distalen Bereich der Nut 68 anschließenden zweiten proximalen Bereich 116 der Nut 68 laufen die Seitenkanten der Nut 68 spitzer aufeinander zu. In dem ersten distalen Bereich 114 der Nut 68 bilden deren Seitenkanten Schneidkanten. In dem zweiten proximalen Bereich 116 der Nut 68 sind deren Seitenkanten und der Nutboden nicht schneidend ausgebildet. Dieser zweite proximale Bereich 116 der Nut 32 dient zur Aufnahme eines Fadens 37.

Zur Befestigung des Nadelhalters 42 in der an dem Schaft 2 des chirurgischen Nahtinstruments ausgebildeten Ausnehmung 40 weist der Nadelhalter 42 im Bereich seines proximalen Endes ein Langloch 48 auf, das schräg zur Längsausdehnung des Nadelhalters 42 ausgerichtet ist. Die Achse des Langlochs 48 verläuft normal zur Anlagefläche des Nadelhalters 42 an dem Schaft 2. An der von der Nut 44 abgewandten Außenseite des Nadelhalters 42 ist um das Langloch 48 ein kreisförmiger Einstich 50 ausgebildet, dessen Durchmesser der Länge des Langlochs 48 entspricht. Korrespondierend zu dem an dem Nadelhalter 42 ausgebildeten Langloch 48 mit Einstich 50 ist an dem Schaft 2 des chirurgischen Nahtinstruments im Bereich der Ausnehmung 40 ein Vorsprung 52 angeordnet, der sich bezogen auf die Längsachse A des Schaftes 2 radial nach außen erstreckt. An seinem Ende weist der Vorsprung 52 eine Erweiterung bzw. Auskragung 54 auf, die sich in Längsrichtung A des Schafts 2 in entgegengesetzte Richtungen nach außen erstreckt. Die Erweiterung 54 weist zu dem Langloch 48 eine im Wesentlichen komplementäre Form und Größe auf.

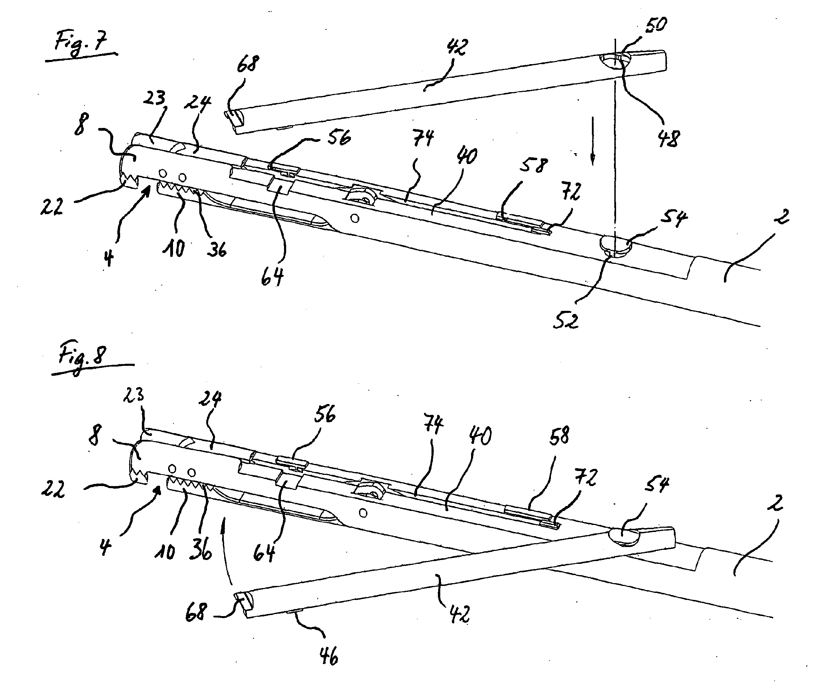

Die Befestigung des Nadelhalters 42 in der Ausnehmung 40 wird insbesondere aus den Fig. 7 und 8 deutlich. Zunächst wird der Nadelhalter 42 so auf die Ausnehmung 40 aufgelegt, dass der Vorsprung 52 mit der daran ausgebildeten Erweiterung 54 in das Langloch 48 eingreift. Wegen der schräg zur Längsausdehnung des Nadelhalters 42 ausgerichteten Anordnung des Langlochs 48 ist der Nadelhalter 42 hierbei schräg zur Längsachse A des Schaftes 2 auf die Ausnehmung 40 aufzulegen. Anschließend ist der Nadelhalter 42 in Richtung des Schaftes 2 zu verschwenken bis er mit dem Schaft 2 fluchtet, so dass die Längsachse des Nadelhalters 42 parallel zur Längsachse A des Schaftes 2 ausgerichtet ist. In dieser Stellung hintergreift die Erweiterung 54 des Vorsprungs 52 den Nadelhalter 42 im Berreich des an dem Langloch 48 ausgebildeten Einstichs 50 und legt den Nadelhalter 42 so in Richtung der Längsausdehnung des Vorsprungs 52, d. h. in radialer Richtung zu der Längsachse A, des Schaftes 2 ausgerichtet, bajonettverschlussartig fest. Zur Festlegung des Nadelhalters 42 an seinem distalen Ende erstreckt sich das proximale Ende des Nadelführungsbauteils 24 über die Ausnehmung 40 des Schafts 2 hinweg, wobei es einen Schlitz 66 bildet. Korrespondierend zu diesem Schlitz 66 ist an dem distalen Ende des Nadelhalters 42 an dessen Außenseite ein Absatz 68 ausgebildet, der in der mit dem Schaft 2 fluchtenden Schwenkstellung des Nadelhalters 42 in den Schlitz 66 eingreift und das Nadelführungsbauteil formschlüssig übergreift.

Zur Begrenzung des Verschwenkweges des Nadelhalters 42 relativ zu dem Schaft 2 sind im Bereich der Ausnehmung 40 zwei Vorsprünge 56 und 58 ausgebildet, die mit zwei an dem Nadelhalter 42 an die Nut 44 angrenzenden Ausnehmungen 60 und 62 in Eingriff bringbar sind. Zur Aufnahme des die Nut 44 des Nadelhalters 42 überdeckenden Bauteils 46 ist in der Ausnehmung 40 ein Einstich 64 eingearbeitet.

Für die Betätigung der in dem Nadelhalter 42 angeordneten Nadel 32 ist eine Betätigungsstange 70 vorgesehen. Diese Betätigungsstange 70 ist in der Nut 14 des Schafts 2 direkt neben der Betätigungsstange 12 bewegungsgeführt. An dem distalen Ende der Betätigungsstange 70 ist ein Nutstein 72 angeordnet, der sich in eine Richtung quer zur Längsausdehnung der Betätigungsstange 70 über diese hinauserstreckt und so einen Vorsprung bildet. Der Nutstein 72 ist in einem an der Ausnehmung 40 ausgebildeten Längsschlitz 74 geführt, wobei der von dem Nutstein 72 gebildete Vorsprung in die Ausnehmung 40 hinein auskragt. Die Bewegungskopplung zwischen Betätigungsstange 70 und Nadel 32 erfolgt dadurch, dass der Nutstein 72 mit einer im Bereich des distalen Endes an der Nadel 32 an dessen Längsseite angeordneten Ausnehmung 75 in Eingriff gebracht wird. Hierdurch wird durch entsprechendes Verschieben der Betätigungsstange 70 ein Vor- und Zurückbewegen der Nadel 32 möglich.

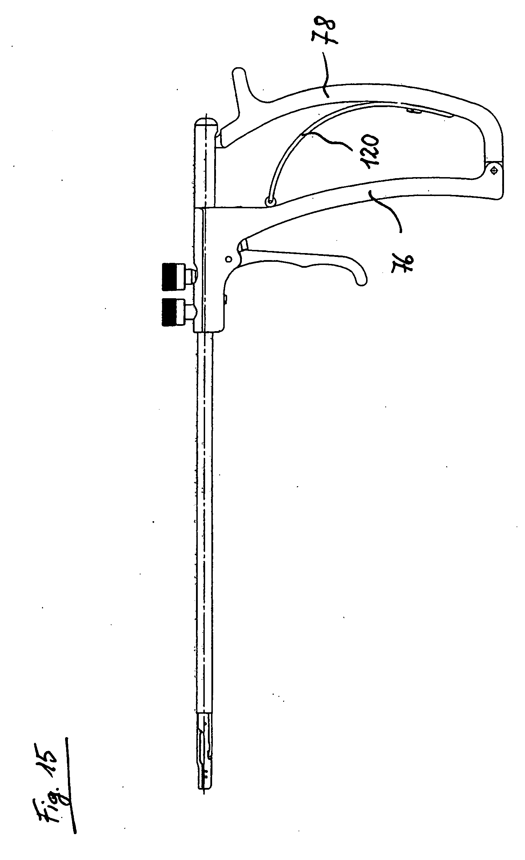

Die an dem proximalen Ende des Schafts 2 angeordnete Handhabe 6 weist einen zweiteiligen Griff auf, der von einem feststehenden ersten Griffteil 76 gebildet wird, an dessen von dem Schaft 2 beabstandeten Ende ein schwenkbares Griffteil 78 über einen Stift 80 angelenkt ist. Das Griffteil 78 ist proximalseitig des Griffteils 76 angeordnet. An dem proximalen Ende der Betätigungsstange 70 ist mittels einer Schraube 82 ein Schieber 84 befestigt. Der Schieber 84 ist in einem Bereich der Handhabe 6 angeordnet, in dem diese eine proximale Verlängerung des Schaftes 2 bildet. In diesem Bereich stützt sich der Schieber 84 an einer distalseitig des Schiebers 84 angeordneten Schraubenfeder 86 ab, wobei in einer alternativen, in Fig. 15 dargestellten Ausgestaltung vorgesehen ist, dass sich der Schieber 84 über das Griffteil 78 an einer zwischen den Griffteilen 78 und 76 angeordneten Blattfeder 120 abstützt. An dem Schieber 84 ist ein quer zur Längsachse A des Schaftes 2 verlaufender Schlitz 88 ausgebildet. In diesen Schlitz 88 greift ein an dem freien Ende des Griffteils 78 angebrachter Stift 91 ein. Auf diese Weise ist das Griffteil 78 über den Schieber 84 mit der Betätigungsstange 70 zum Bewegen der Nadel 32 bewegungsgekoppelt.

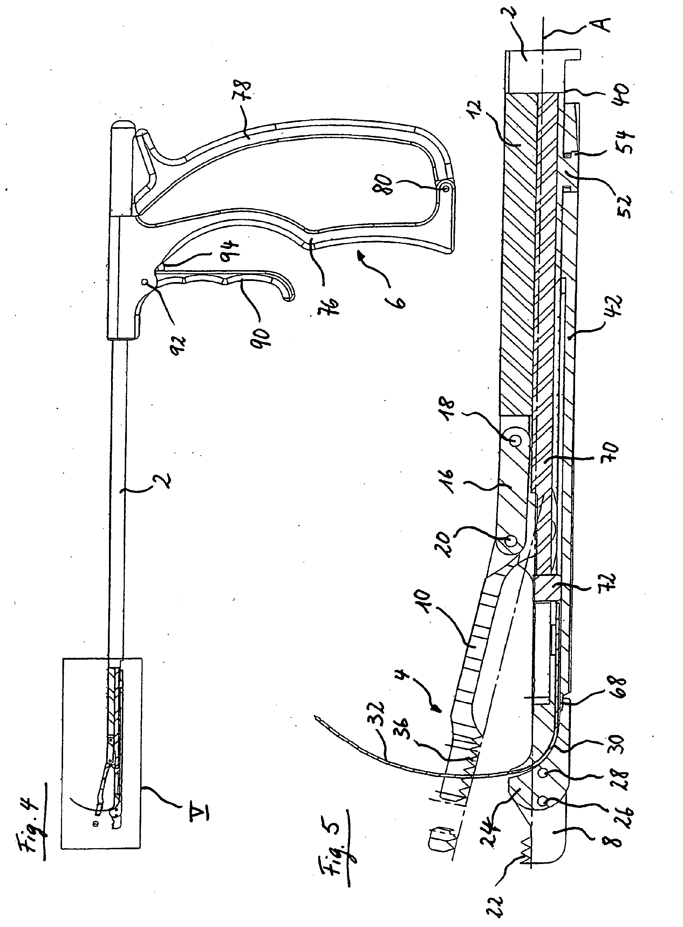

Indem das Griffteil 78 in Richtung des Griffteils 76 verschwenkt wird, wird die Betätigungsstange 70 und damit eine in dem Nadelhalter 42 angeordnete Nadel 32 in distaler Richtung gegen die Federkraft der Schraubenfeder 86 verschoben. Hierdurch gelangt die Nadel 32, wie in Fig. 5 dargestellt durch den an dem Nadelführungsbauteil 24 angeordneten Führungskanal 30 und anschließend durch die an dem Maulteil 10 ausgebildete Durchbrechung 34. Durch einfaches Loslassen des Griffteils 78 wird die Nadel 32 mit der Betätigungsstange 70 durch die Entspannung der Schraubenfeder 86 wieder in den Nadelhalter 42 zurückbewegt.

Distalseitig des Griffteils 76 ist an dem die Verlängerung des Schaftes 2 bildenden Bereich der Handhabe 6 ein Betätigungshebel 90 über einen Stift 92 angelenkt. Der Betätigungshebel 90 stützt sich an seiner dem Griffteil 76 zugewandten Seite an einem Stift 94 ab, der auf einer in dem Griffteil 76 angeordneten Schraubenfeder 96 gelagert ist. An dem in der Handhabe 6 angeordneten Ende des Betätigungshebels 90 ist ein Mitnehmer 98 ausgebildet der in einer an dem proximalen Ende der Betätigungsstange 12 angeordneten Ausnehmung in Eingriff ist. Wird der Betätigungshebel 90 in Richtung des Griffteils 76 gegen die Federkraft der Schraubenfeder 96 verschwenkt, wird die Betätigungsstange 12 in distaler Richtung verschoben und hierdurch das Maulteil 10 des Zangenmauls 4 in eine öffnende Stellung verschwenkt. Durch Loslassen des Betätigungshebels 90 wird die Betätigungsstange 12 unter Entspannung der Schraubenfeder 96 wieder proximalwärts verschoben und das Zangenmaul 4 geschlossen.

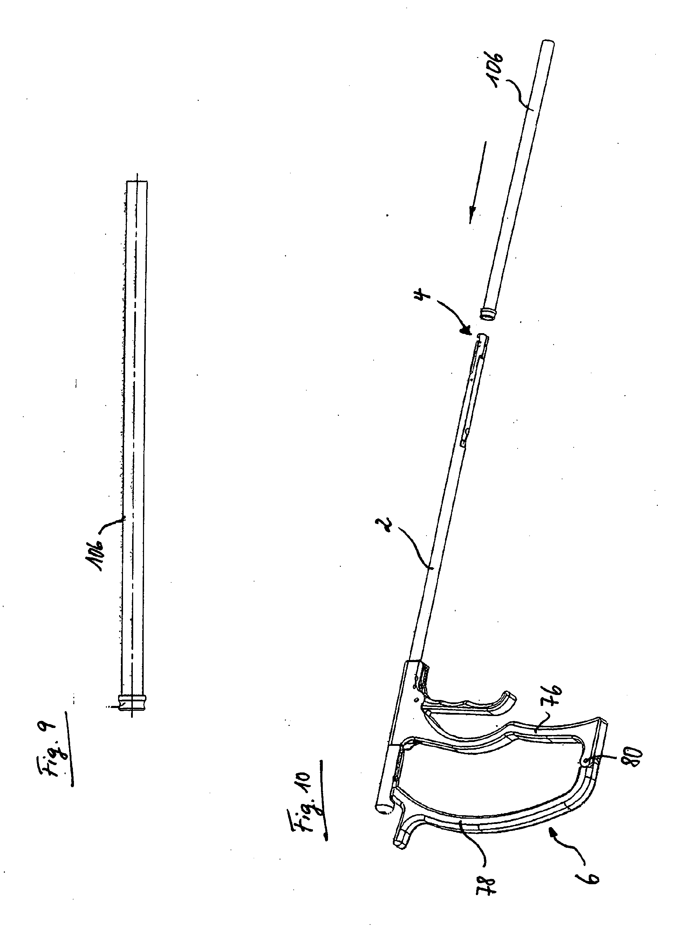

Distalseitig des Betätigungshebels 90 ist an dem die Verlängerung des Schaftes 2 bildenden Bereich der Handhabe 6 eine Feder 100 angeordnet, die mit einer Schraube 102 festgelegt ist. Diastalseitig der Feder 100 stützt sich an dieser eine Kugel 104 ab, die einen Rastkörper einer Kugelrastverbindung bildet. Mittels dieser Kugelrastverbindung ist ein in den Fig. 9 und 10 dargestellter Außenschaft 106, der im zusammengebauten Zustand des chirurgischen Nahtinstruments den Schaft 2 umgibt, an der Handhabe 6 festlegbar. Hierzu weist der Außenschaft 106 an seinem proximalen Ende eine Ringnut 108 auf, in die die Kugel 104 eingreifen kann.

Nachfolgend wird die Vorgehensweise mit dem erfindungsgemäßen chirurgischen Nahtinstrument anhand der Fig. 11 bis 14 erläutert.

Bei geöffnetem Zangenmaul 4 wird ein Faden 37 schlaufenförmig in die eine Fadenführung bildende Nut 33 des Nadelführungsbauteils 24 eingelegt (Fig.11). Der Faden 37 liegt nun direkt oberhalb der Mündung 35 des durch das Nadelführungsbauteils 24 verlaufenden Führungskanals 30. Die Nadel 32 befindet sich zu diesem Zeitpunkt noch in dem Nadelhalter 42 (Fig. 5,6). Anschließend wird das zu vernähende Gewebe 110 mit dem Zangenmaul 4 des chirurgischen Nahtinstruments ergriffen (Fig. 12).

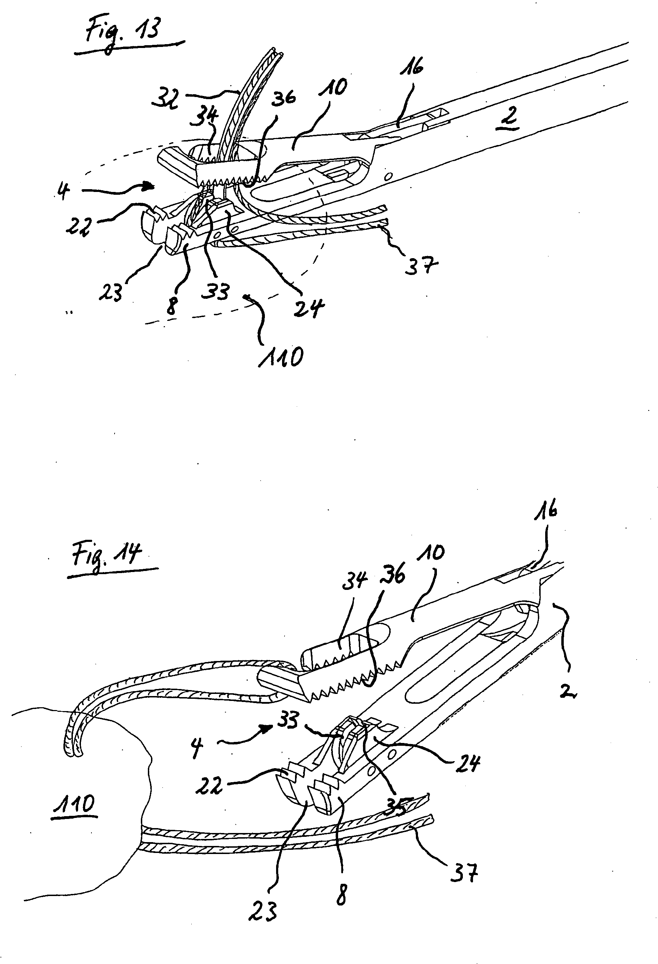

Daraufhin wird die Nadel 32 in distaler Richtung verschoben, wobei der Faden 37 beim Austreten der Nadel 32 aus der Mündung des Führungskanals 30 von der Nadel 32 aufgenommen wird. Hierbei kommt der Faden in dem zweiten proximalseitig gelegenen Bereich der an der Schneidkante der Nadel ausgebildeten Nut 68 zur Anlage, der nicht schneidend ausgebildet ist. Zusammen mit der Nadel 32 wird der Faden 37 nun durch das Gewebe 110 geführt (Fig. 13). Anschließend wird die Nadel 32 ohne den Faden 37 in proximaler Richtung durch die Feder 86, 120 zurück in den Nadelhalter 42 verschoben. Die Greifstellung des Zangenmauls 4 wird gelöst und die von dem durch das Gewebe geführten Faden 37 gebildete Schlaufe mit Hilfe des schwenkbaren Maulteils 10 vergrößert (Fig. 14)

### Bezugszeichenliste

- 2: Schaft
- 4: Zangenmaul
- 6: Handhabe
- 8: Maulteil
- 10: Maulteil
- 12: Betätigungsstange
- 14: Nut
- 16: Gelenkteil
- 18: Stift
- 20: Stift
- 22: Haltebereich
- 23: Schlitz
- 24: Nadelführungsbauteil
- 26: Stift
- 28: Stift
- 30: Führungskanal
- 32: Nadel
- 33: Nut
- 34: Durchbrechung
- 35: Mündung
- 36: Haltebereich
- 37: Faden
- 38: Schlitz
- 40: Ausnehmung
- 42: Nadelhalter
- 44: Nut
- 46: Steg
- 48: Langloch
- 50: Einstich
- 52: Vorsprung
- 54: Erweiterung, Auskragung
- 56: Vorsprung
- 58: Vorsprung
- 60: Ausnehmung
- 62: Ausnehmung
- 64: Einstich
- 66: Schlitz
- 68: Nut
- 70: Betätigungsstange
- 72: Nutstein
- 74: Längsschlitz
- 75: Ausnehmung
- 76: Griffteil
- 78: Griffteil
- 80: Stift
- 82: Schraube
- 84: Schieber
- 86: Feder
- 88: Schlitz
- 90: Betätigungshebel
- 91: Stift
- 92: Stift
- 94: Stift
- 96: Schraubenfeder
- 98: Mitnehmer
- 100: Feder
- 102: Schraube
- 104: Kugel
- 106: Außenschaft
- 108: Ringnut
- 110: Gewebe
- 112: Schneidkante
- 114: Bereich
- 116: Bereich
- 120: Blattfeder

- A: Längsachse

## Patentansprüche

1. Chirurgisches Nahtinstrument mit einem Schaft (2), an dessen distalen Ende ein Zangenmaul (4) ausgebildet ist, und mit einer Nadelführung (30) durch das Zangenmaul (4), **dadurch gekennzeichnet, dass** an dem Schaft (2) proximalseitig an die Nadelführung (30) anschließend eine Aufnahme (40) zum lösbaren Befestigen eines Nadelhalters (42) ausgebildet ist.

2. Chirurgisches Nahtinstrument nach Anspruch 1, **dadurch gekennzeichnet, dass** ein Nadelhalter (42) vorgesehen ist, welcher eine Nadelführung (44) zur Aufnahme und Führung einer Nadel (32) aufweist und derart lösbar an der Aufnahme (40) des Schaftes (2) befestigbar ist, dass sich die Nadelführung (44) im Nadelhalter (42) an die Nadelführung (30) durch das Zangenmaul (4) anschließt, wobei der Nadelhalter (42) bevorzugt einen Teil des Schaftes (2) bildet.

3. Chirurgisches Nahtinstrument nach Anspruch 2, **dadurch gekennzeichnet, dass** an dem Schaft (2) und/oder dem Nadelhalter (42) Befestigungsmittel zum lösbaren Befestigen des Nadelhalters (42) an der Aufnahme (40) an dem Schaft (2) vorgesehen sind, wobei die Befestigungsmittel bevorzugt nach Art eines Bajonettverschlusses ausgestaltet sind.

4. Chirurgisches Nahtinstrument nach einem der vorangehenden Ansprüche, **dadurch gekennzeichnet, dass** an dem Schaft (2) ein quer zu seiner Längsachse (A) ausgerichteter Vorsprung (52) angeordnet ist, dessen Endbereich (54) in zumindest eine Richtung quer zur Längsausdehnung des Vorsprungs (52) erweitert ist.

5. Chirurgisches Nahtinstrument nach einem der vorangehenden Ansprüche, **dadurch gekennzeichnet, dass** an einem Maulteil (8) des Zangenmauls (4) eine Fadenführung (33) ausgebildet ist, die die Nadelführung (30) durch das Zangenmaul (4) kreuzt.

6. Chirurgisches Nahtinstrument nach einem der vorangehenden Ansprüche, **dadurch gekennzeichnet, dass** das Nahtinstrument eine Betätigungsstange (70) aufweist, welche im Bereich der Aufnahme (40) für den Nadelhalter (42) Eingriffsmittel (72) zum I-neingrifftreten mit einer in dem Nadelhalter (42) eingesetzten Nadel (32) aufweist.

7. Nadelhalter (42) für ein chirurgisches Nahtinstrument und insbesondere für ein chirurgisches Nahtinstrument nach einem der Ansprüche 1 bis 6, mit einer Nadelführung (44) zur Aufnahme und Führung einer Nadel (32), **dadurch gekennzeichnet, dass** der Nadelhalter (42) zum lösbaren Befestigen an einer Aufnahme (40) eines Schaftes (2) eines chirurgischen Nahtinstruments ausgebildet ist.

8. Nadelhalter (42) nach Anspruch 7, **dadurch gekennzeichnet, dass** an dem Nadelhalter (42) Befestigungsmittel zum lösbaren Befestigen an einer Aufnahme an dem Schaft eines chirurgischen Nahtinstruments vorgesehen sind, wobei die Befestigungsmittel bevorzugt nach Art eines Bajonettverschlusses ausgestaltet sind.

9. Nadelhalter (42) nach einem der Ansprüche 7 oder 8, **dadurch gekennzeichnet, dass** die Nadelführung in dem Nadelhalter (42) eine sich in Richtung seiner Längsausdehnung erstreckende Nut ist, die an einem distalen Ende des Nadelhalters (42) mündet.

10. Nadelhalter (42) nach Anspruch 9, **dadurch gekennzeichnet, dass** Mittel vorgesehen sind, die die offene Seite der Nut zumindest abschnittsweise überdecken.

11. Nadelhalter (42) nach einem der Ansprüche 8 oder 10, **dadurch gekennzeichnet, dass** im Bereich eines proximalen Endes des Nadelhalters (42) ein vorzugsweise schräg zur Längsausdehnung des Nadelhalters (42) ausgerichtetes Langloch angeordnet ist.

12. Nadelhalter (42) nach einem der Ansprüche 7 bis 11, **dadurch gekennzeichnet, dass** der Nadelhalter (42) in einem chirurgischen Nahtinstrument gemäß einem der Ansprüche 1 bis 6 angeordnet ist.

13. Nadel (32) für ein chirurgisches Nahtinstrument gemäß einem der Ansprüche 1 bis 6 und/oder für einen Nadelhalter (42) gemäß einem der Ansprüche 7 bis 12, welche von einem Materialstreifen gebildet wird, der ein distales und ein proximales Ende aufweist, wobei an dem distalen Ende eine Schneidkante (112) ausgebildet ist, **dadurch gekennzeichnet, dass** an der Schneidkante (112) eine sich quer zur Schneidkante (112) proximalwärts erstreckende Ausnehmung (68) ausgebildet ist, welche eine proximalseitige nicht schneidend ausgebildete Kante aufweist.

14. Nadel (32) nach Anspruch 13, **dadurch gekennzeichnet, dass** an der Schneidkante (112) eine Nut (68) ausgebildet ist, die die Schneidkante (112) in zwei voneinander beabstandete Schneidkantenabschnitte teilt, wobei der Nutboden nicht schneidend ausgebildet ist.

15. Nadel (32) nach Anspruch 14, **dadurch gekennzeichnet, dass** sich die Nutbreite in proximaler Richtung verjüngt.

16. Nadel (32) nach einem der Ansprüche 14 oder 15, **dadurch gekennzeichnet, dass** sich die Nutbreite in proximaler Richtung zweistufig verjüngt, wobei die beiden Seitenwände der Nut (68) in einem ersten distalen Abschnitt (114) der Nut (68) jeweils eine Schneidkante bilden und ein proximaler Abschnitt (116) der Nut (68) eine nicht schneidende Kante aufweist und eine Aufnahme für einen Faden (37) bildet.

17. Nadel (32) nach einem der Ansprüche 13 bis 16, **dadurch gekennzeichnet, dass** die Nadel (32) Mittel aufweist, die mit einer Betätigungsstange (70) eines chirurgischen Nahtinstruments in Eingriff treten können.

18. Nadel (32) nach einem der Ansprüche 13 bis 17, **dadurch gekennzeichnet, dass** die Nadel (32) im Bereich ihres proximalen Endes an einer Längsseite eine sich quer zur Längsausdehnung der Nadel (32) erstreckende Ausnehmung (75) aufweist.

19. Nadel (32) nach einem der Ansprüche 13 bis 18, **dadurch gekennzeichnet, dass** die Nadel (32) in einer Nadelführung (44) eines Nadelhalters (42) gemäß einem der Ansprüche 7 bis 12 angeordnet ist.

## Claims

1. A surgical suture instrument with a shank (2), on whose distal end a forceps jaw (4) is formed, and with a needle guide (30) through the forceps jaw (4), **characterised in that** a receiver (40) for the releasable fastening of a needle holder (42) is formed on the shank (2) connecting to the needle guide (30) on the proximal side.

2. A surgical suture instrument according to claim 1, **characterised in that** a needle holder (42) is provided, which comprises a needle guide (44) for receiving and guiding a needle (32), and may be releasably fastened on the receiver (40) of the shank (2), in a manner such that the needle guide (44) in the needle holder (42) connects to the needle guide (30) through the forceps jaw (4), wherein the needle holder (42) preferably forms a part of the shank (2).

3. A surgical suture instrument according to claim 2, **characterised in that** fastening means for the releasable fastening of the needle holder (42) on the receiver (40) on the shank (2), are provided on the shank (2) and/or on the needle holder (42), wherein the fastening means are preferably designed in the manner of a bayonet closure.

4. A surgical suture instrument according to one of the preceding claims, **characterised in that** a projection (52) aligned transversely to its longitudinal axis (A) of the shank (2) is arranged on the shank (2), and whose end region (54) is widened in at least one direction transversely to the longitudinal extension of the projection (52).

5. A surgical suture instrument according to one of the preceding claims, **characterised in that** a thread guide (33) is formed on a jaw part (8) of the forceps jaw (4), and this crosses the needle guide (30) through the forceps jaw (4).

6. A surgical suture instrument according to one of the preceding claims, **characterised in that** the suture instrument comprises an actuation rod (70), which in the region of the receiver (40) for the needle holder (42) comprises engagement means (72) for engagement with a needle (32) inserted in the needle holder (42).

7. A needle holder (42) for a surgical suture instrument and in particular for a surgical suture instrument according to one of the claims 1 to 6, with a needle guide (44) for receiving and guiding a needle (32), **characterised in that** the needle holder (42) is designed for the releasable fastening on a receiver (40) of a shank (2) of a surgical suture instrument.

8. A needle holder (42) according to claim 7, **characterised in that** fastening means for the releasable fastening on a receiver on the shank of a surgical suture instrument are provided on the needle holder (42), wherein the fastening means are preferably designed in the manner of a bayonet closure.

9. A needle holder (42) according to one of the claims 7 or 8, **characterised in that** the needle guide in the needle holder (42) is a groove which extends in the direction of its longitudinal extension and which runs out at the distal end of the needle holder (42).

10. A needle holder (42) according to claim 9, **characterised in that** means are provided which cover the open side of the groove, at least in sections.

11. A needle holder (42) according to one of the claims 8 or 10, **characterised in that** an elongate hole preferably directed obliquely to the longitudinal extension of the needle holder (42), is arranged in the region of a proximal end of the needle holder (42).

12. A needle holder (42) according to one of the claims 7 to 11, **characterised in that** the needle holder (42) is arranged in a surgical suture instrument according to one of the claims 1 to 6.

13. A needle (32), in particular for a surgical suture instrument according to one of the claims 1 to 6 and/or for a needle holder (42) according to one of the claims 7 to 12, which is formed by a material strip which has a distal and proximal end, wherein a cutting edge (112) is formed at the distal end, **characterised in that** a recess (68) which extends proximally, transversally to the cutting edge (112) and which has an edge on the proximal side which is not designed in a cutting manner, is formed on the cutting edge (112).

14. A needle (32) according to claim 13, **characterised in that** a groove (68) which divides the cutting edge (112) into two cutting edge sections distanced to one another, is formed on the cutting edge (112), wherein the groove base is designed in a non-cutting manner.

15. A needle (32) according to claim 14, **characterised in that** the groove width tapers in the proximal direction.

16. A needle (32) according to one of the preceding claims 14 or 15, **characterised in that** the groove width tapers in a two-stage manner in the proximal direction, wherein the two side walls of the groove (68) in a first distal section (114) of the groove (68) in each case form a cutting edge, and a proximal section (116) of the groove (68) comprises a non-cutting edge and forms a receiver for a thread (37).

17. A needle (32) according to one of the claims 13 to 16, **characterised in that** the needle (32) comprises means which may come into engagement with an actuation rod (70) of a surgical suture instrument.

18. A needle (32) according to one of the claims 13 to 17, **characterised in that** the needle (32) in a region of its proximal end, on a longitudinal side, comprises a recess (75) extending transversely to the longitudinal extension of the needle (32).

19. A needle (32) according to one of the claims 13 to 18, **characterised in that** the needle (32) is arranged in a needle guide (44) of a needle holder (42) according to one of the claims 7 to 12.

## Revendications

1. Instrument de suture chirurgical comportant une tige (2), sur l'extrémité distale de laquelle est formée une mâchoire de pince (4), et comportant un guide-aiguille (30) à travers la mâchoire de pince (4), **caractérisé en ce qu'**au niveau de la tige (2), du côté proximal et raccordé au guide-aiguille (30), est formé un logement (40) destiné à la fixation amovible d'un porte-aiguille (42).

2. Instrument de suture chirurgical selon la revendication 1, **caractérisé en ce qu'**un porte-aiguille (42) est prévu, lequel présente un guide-aiguille (44) destiné à loger et guider une aiguille (32) et peut être fixé de manière amovible au logement (40) de la tige (2) de telle sorte que le guide-aiguille (44) du porte-aiguille (42) se raccorde au guide-aiguille (30) à travers la mâchoire (4), le porte-aiguille (42) formant de préférence une partie de la tige (2).

3. Instrument de suture chirurgical selon la revendication 2, **caractérisé en ce qu'**au niveau de la tige (2) et/ou du porte-aiguille (42) sont prévus des moyens de fixation destinés à la fixation amovible du porte-aiguille (42) sur le logement (40) au niveau de la tige (2), les moyens de fixation étant réalisés de préférence selon le type d'un emboîtement à baïonnette.

4. Instrument de suture chirurgical selon l'une des revendications précédentes, **caractérisé en ce qu'**au niveau de la tige (2) est agencée une saillie (52) orientée transversalement par rapport à son axe longitudinal (A), saillie dont la zone d'extrémité (54) est élargie dans au moins une direction transversalement à l'extension longitudinale de la saillie (52).

5. Instrument de suture chirurgical selon l'une des revendications précédentes, **caractérisé en ce qu'**au niveau d'un élément de mâchoire (8) de la mâchoire de pince (4) est formé un guide-fil (33) qui croise le guide-aiguille (30) à travers la mâchoire de pince (4).

6. Instrument de suture chirurgical selon l'une des revendications précédentes, **caractérisé en ce que** l'instrument de suture présente une tige d'actionnement (70), laquelle présente dans la zone du logement (40) du porte-aiguille (42) des moyens d'engagement (72) permettant l'engagement avec une aiguille (32) insérée dans le porte-aiguille (42).

7. Porte-aiguille (42) pour instrument de suture chirurgical et en particulier pour instrument de suture chirurgical selon l'une des revendications 1 à 6, comportant un guide-aiguille (44) destiné à loger et guider une aiguille (32), **caractérisé en ce que** le porte-aiguille (42) est formé pour la fixation amovible d'un instrument de suture chirurgical au niveau d'un logement (40) d'une tige (2).

8. Porte-aiguille (42) selon la revendication 7, **caractérisé en ce qu'**au niveau du porte-aiguille (42) sont prévus des moyens de fixation destinés à la fixation amovible au niveau d'un logement sur la tige d'un instrument de suture chirurgical, les moyens de fixation étant de préférence réalisés selon le type d'un emboîtement à baïonnette.

9. Porte-aiguille (42) selon l'une des revendications 7 ou 8, **caractérisé en ce que** le guide-aiguille du porte-aiguille (42) est une rainure s'étendant dans la direction de son extension longitudinale, laquelle rainure débouche au niveau d'une extrémité distale du porte-aiguille (42).

10. Porte-aiguille (42) selon la revendication 9, **caractérisé en ce que** sont prévus des moyens qui recouvrent au moins partiellement le côté ouvert de la rainure.

11. Porte-aiguille (42) selon l'une des revendications 8 ou 10, **caractérisé en ce que** dans la zone d'une extrémité proximale du porte-aiguille (42) est agencé un orifice oblong orienté de préférence de manière inclinée par rapport à l'extension longitudinale du porte-aiguille (42).

12. Porte-aiguille (42) selon l'une des revendications 7 à 11, **caractérisé en ce que** le porte-aiguille (42) est agencé dans un instrument de suture chirurgical selon l'une des revendications 1 à 6.

13. Aiguille (32) pour instrument de suture chirurgical selon l'une des revendications 1 à 6 et/ou pour porte-aiguille (42) selon l'une des revendications 7 à 12, laquelle est formée par une bande de matériau qui présente une extrémité distale et une extrémité proximale, une arête coupante (112) étant formée au niveau de l'extrémité distale, **caractérisée en ce qu'**au niveau de l'arête coupante (112) est formé un évidement (68) s'étendant vers l'extrémité proximale transversalement par rapport à l'arête coupante (112), lequel évidement présente une arête exécutée côté proximal de manière à ne pas couper.

14. Aiguille (32) selon la revendication 13, **caractérisée en ce que** sur l'arête coupante (112) est formée une rainure (68) qui partage l'arête coupante (112) en deux sections d'arête coupante à distance l'une de l'autre, le fond de la rainure étant configuré de manière à ne pas couper.

15. Aiguille (32) selon la revendication 14, **caractérisée en ce que** la largeur de la rainure se rétrécit dans la direction proximale.

16. Aiguille (32) selon l'une des revendications 14 ou 15, **caractérisée en ce que** la largeur de la rainure se rétrécit en deux phases dans la direction proximale, les deux parois latérales de la rainure (68) formant dans une première section distale (114) de la rainure (68) respectivement une arête coupante, et une section proximale (116) de la rainure (68) présentant une arête non coupante et formant un logement de fil (37).

17. Aiguille (32) selon l'une des revendications 13 à 16, **caractérisée en ce que** l'aiguille (32) présente des moyens qui peuvent s'engager avec une tige d'actionnement (70) d'un instrument de suture chirurgical.

18. Aiguille (32) selon l'une des revendications 13 à 17, **caractérisée en ce que** l'aiguille (32) présente dans la zone de son extrémité proximale, au niveau d'un côté longitudinal, un évidement (75) s'étendant transversalement par rapport à l'extension longitudinale de l'aiguille (32).

19. Aiguille (32) selon l'une des revendications 13 à 18, **caractérisée en ce que** l'aiguille (32) est agencée dans un guide-aiguille (44) d'un porte-aiguille (42) selon l'une des revendications 7 à 12.
